# EUROPEAN PATENT APPLICATION

(11) **EP 1 852 117 A1**
(43) Date of publication of application: **07.11.2007**
(21) Application number: 06714366.9
(22) Date of filing: 23.02.2006
(51) Int. Cl.: A61K 31/439

(54) **PHARMACEUTICAL AGENT COMPRISING SOLIFENACIN**

(30) Priority: 25.02.2005 JP 2005050370
(71) Applicant: Astellas Pharma Inc., Tokyo 103-8411 (JP)
(72) Inventor: IKEDA, Ken, 2-chome, Chuo-ku Tokyo, 1038411 (JP); SATO, Shuichi, 2-chome, Chuo-ku Tokyo, 1038411 (JP); SUZUKI, Masanori, 2-chome, Chuo-ku Tokyo, 1038411 (JP); OHTAKE, Akiyoshi, 2-chome, Chuo-ku Tokyo, 1038411 (JP); IKEDA, Yasushi, 2-chome, Chuo-ku Tokyo, 1038411 (JP); YASUKAWA, Kenji, 2-chome, Chuo-ku Tokyo, 1038411 (JP)
(74) Representative: Bates, Philip Ian
(86) International application number: PCT/JP2006/303226
(87) International publication number: WO 2006/090759

(57) **Abstract**

An agent for ameliorating urinary urgency, pollakiuria and urinary incontinence cause by neurogenic bladder including cerebrovascular disorder or a neurodegenerative disease such as spinal cord compression or injury or the like associated with cerebrosclerosis, brain and spinal cord injury caused by trauma, multiple sclerosis, Parkinson's disease, congenital abnormality in the morphogenesis of the nervous system, peripheral nervous system disorder and various disorders of the spinal cord (i.e., fracture, cervical and lumbar spondylopathy, spondylosis deformans, spondylolisthesis, spinal canal stenosis, disk herniation or the like, the agent comprising 5 to 10 mg of solifenacin succinate or the combination of 5 to 10 mg of solifenacin succinate with an equimolar amount of solifenacin or a pharmaceutical acceptable salt thereof as the active ingredient.

## Description

### Technical Field

The present invention relates an agent for improvement of urinary urgency, pollakiuria and urinary incontinence, particularly urinary urgency and pollakiuria associated with neurogenic bladder that comprises solifenacin or a salt thereof.

### Background Art

Solifenacin has the structure shown below and solifenacin or a salt thereof is known as a muscarinic M₃ receptor antagonist (Patent Document 1, Non-patent Document 1, Non-patent Document 2 and Non-patent Document 3). Its chemical name is (3R)-quinuclidin-3-yl (1S)-1-phenyl-1,2,3,4-tetrahydroisoquinoline-2-carboxylate.

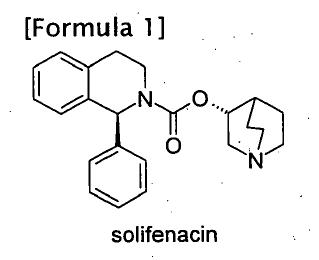

Solifenacin or a salt thereof is reported to be useful for the treatment of various diseases in which the M₃ receptor is involved (Patent Document 1) and to be effective in interstitial cystitis (Patent Document 2), for relaxation of the ciliary muscle (Patent Document 3) and in irritable bowel syndrome (Non-patent Document 4). Solifenacin succinate, a succinate salt of solifenacin, has been confirmed to be effective in urinary urgency, pollakiuria and urinary incontinence in unstable bladder in a patient population having no neurological disease, urinary tract infections, urinary calculi, lumbar irradiation history and a tumor in the pelvic organ that may cause detrusor overactivity (Patent Document 5). On the other hand, a condition in which urinary urgency, pollakiuria and urinary incontinence occur in the absence of such causative diseases is defined as overactive bladder (Non-patent Document 6). According to the definition in "The Standardization of Terminology of Lower Urinary Tract Function" (Non-patent Document 7), urinary urgency is "complaint of sudden, compelling desire to pass urine that is difficult to defer", pollakiuria is "patient complaint that number of urination is too high" and urinary incontinence is "patient complaint that urine leaks involuntarily".

In the above Patent Document 1, solifenacin or a salt thereof is disclosed as a muscarinic M₃ antagonist, and there are mentioned urologic diseases such as urinary incontinence, pollakiuria and the like in nervous pollakiuria, neurogenic bladder, nocturnal enuresis, unstable bladder, cystospasm, chronic cystitis and the like; respiratory diseases such as chronic obstructive pulmonary disease, chronic bronchitis, asthma, rhinitis and the like; and gastrointestinal diseases such irritable colon syndrome, spasmodic colitis, diverticulitis and the like as diseases for which solifenacin and its salts may be effective as a prophylactic or therapeutic agent. However, the efficacy against neurogenic bladder is not disclosed with specific data. Especially, improvement of urinary urgency in neurogenic bladder is not described or suggested.

Generally, "neurogenic bladder" refers to a condition in which abnormal bladder function is observed in patients with neurological diseases such as cerebral infarction, multiple sclerosis, spinal cord injury, parkinsonism and congenital anomaly of the nerve system (Non-patent Document 8, Non-patent Document 9 and Non-patent Document 10). Neurogenic bladder associated with urinary urgency, pollakiuria and urinary incontinence is diagnosed as clearly distinguished from unstable bladder, which exhibits urinary urgency, pollakiuria and urinary incontinence in the absence of a causal disease such as neurological disease, and has sensitivity to drugs different from that of unstable bladder (Non-patent Document 10 and Non-patent Document 11).

Currently, as antimuscarinic agents, oxybutynin, tolterodine, propiverine, darifenacin and the like are mainly known in addition to solifenacin.
Among them, oxybutynin is reported to have therapeutic efficacy against urinary urgency, pollakiuria and urinary incontinence in neurogenic bladder associated with multiple sclerosis, spinal cord injury and Parkinson's disease (Non-patent Document 11). However, the dose of oxybutynin required for the treatment of these symptoms is reported to be higher than the dose required for the treatment of symptoms of urinary urgency, pollakiuria and urinary incontinence in unstable bladder (Non-patent Document 11). In addition, efficacy of oxybutynin in patients with cerebrovascular disorder is reported to be unknown (Non-patent Document 12).

As for tolterodine, a document describes that tolterodine is used for the treatment of urinary incontinence or detrusor overactivity in neurogenic bladder attributable to cerebrovascular accidents, but there is no specific description about an improving effect on urinary urgency, pollakiuria and urinary incontinence (Non-patent Document 12). Further, it is reported that, in a clinical trial of tolterodine in a population of paitents with urinary urgency, pollakiuria and urinary incontinence associated with neurogenic bladder, efficacy against pollakiuria and urinary incontinence was confirmed, but efficacy against urinary urgency was reported not able to be evaluated (Non-patent Document 13). In addition, in the results of a clinical trial of tolterodine using a population of patients with urinary urgency, pollakiuria and urinary incontinence attributable to unstable bladder or neurogenic bladder, efficacy against pollakiuria and urinary incontinence is described, but efficacy against urinary urgency is not described (Non-patent Document 14). On the other hand, although it is suggested that tolterodine is effective in urinary inconsistence in patients with neurogenic bladder who cannot urinate by oneself, but a dose higher than the dose required for the treatment of unstable bladder is required to obtain the optimum effect (Non-patent Document 15), and no effect on urinary urgency or pollakiuria is reported.

As for propiverine, according to its package insert in England, the indications are "treatment of urinary incontinence, urinary urgency, and pollakiuria in patients with overactive bladder. (that is, unstable bladder) or neurogenic bladder." It is generally recommended to administer a coated preparation containing 15 mg of propiverine hydrochloride 2 times daily. For neurogenic bladder, however, it is recommended to administer the same coated preparation 3 times daily. In other words, it is recommended that a higher dose should be administered for the treatment of urinary urgency, pollakiuria and urinary incontinence in neurogenic bladder as compared with a dose for the treatment of these symptoms in unstable bladder.
As for darifenacin, there is no report about therapeutic efficacy against neurogenic bladder.

As described above, as for antimuscarinic agents, although some documents have reported efficacy against urinary urgency, pollakiuria and urinary incontinence in neurogenic bladder, it is suggested or recommended that a higher dose is required to improve symptoms of urinary urgency, pollakiuria and urinary incontinence, especially urinary urgency in neurogenic bladder than the dose required for the treatment of unstable bladder.

On the other hand, solifenacin or a salt thereof is disclosed as an agent that suppresses the capsaicin-sensitive sensory nerve in the above Patent Document 2 and use of dimethylsulfoxide that has an inhibitory effect on the capsaicin-sensitive sensory nerve for interstitial cystitis has been approved by FDA in the U.S. Accordingly, sensory hypersensitivity in the lower urinary tract and/or non-bacterial prostatitis, diseases similar to interstitial cystitis, are mentioned as diseases for which solifenacin or a salt thereof is possibly effective. However, efficacy of solifenacin or a salt thereof against neurogenic bladder has not been described or suggested.

As for the relationship between the capsaicin-sensitive sensory nerve and urinary urgency, pollakiuria and urinary incontinence in neurogenic bladder, it is reported that intravesical injection of the neurotoxins selective for C-fibers capsaicin and resiniferatoxin having a vanilloid receptor stimulating effect is useful for the improvement of urinary urgency, pollakiuria and urinary incontinence in patients with multiple sclerosis or spinal cord injury (Non-patent Document 16 and Non-Patent Document 9). However, both capsaicin and resiniferatoxin have been clinically used only on an trial base and have not yet been used in clinical practice. They are not administered orally.
At the same time, studies of capsaicin and resiniferatoxin, which are expected to be effective in urinary urgency, pollakiuria and urinary incontinence in overactive bladder, that is, unstable bladder, in humans are limited to those in patients with neurogenic bladder and interstitial cystitis (Non-patent Document 9), and no document has reported their usefulness for unstable bladder. The reasons are considered as follows: these drugs are administered from the urethra into the bladder via a catheter; the administration method is invasive; pain occurs at an administration site due to the vanilloid receptor stimulatory effect, and systemic side effects sometimes develop (Non-patent Document 9). Accordingly, the dose of capsaicin and resiniferatoxin required for the treatment of urinary urgency, pollakiuria and urinary incontinence in unstable bladder are unknown. In addition, these drugs are not administered orally.

[Non-patent Document 1] Current Opinion in Central and Peripheral Nervous System, Investigational Drugs, 2000, Vol. 2, No. 3, p. 321-325
[Non-patent Document 2] Drugs of the Future, 1999, Vol. 24, No. 8, p. 871-874
[Non-patent Document 3] Naunyn-Schmiedeberg's Archives of Pharmacology, 2002, Vol. 366, No. 2, p. 97-103
[Non-patent Document 4] Japanese Journal of Pharmacology, 2001, vol. 86, No. 3, p. 281-288
[Non-patent Document 5] BJU International, 2004, Vol. 93, p. 303-310
[Non-patent Document 6] Drugs, 2004, Vol. 64, No. 15, p. 1643-1656
[Non-patent Document 7] Neurology and Urodynamics, 2002, Vol. 21, p. 167-178
[Non-patent Document 8] Revue du Praticien, 1995, Vol. 45, p. 331-335
[Non-patent Document 9] The Journal of Urology, 1999, Vol. 162, p. 3-11
[Non-patent Document 10] Spinal Cord, 2004, Vol. 42, p. 267-272
[Non-patent Document 11] The Journal of Urology, 2004, Vol. 171, p. 749-751
[Non-patent Document 12] The Journal of Urology, 2001, Vol. 165, p. 359-370
[Non-patent Document 13] Neurology and Urodynamics, 1998, Vol. 17, p. 499-512
[Non-patent Document 14] The Journal of Urology, 1999, Vol. 161, p. 1551-1555
[Non-patent Document 15] Journal of Spinal Cord Medicine, 2004, Vol. 27, No. 3, p. 214-218
[Non-patent Document 16] The Journal of Urology, 2004, Vol. 171, p. 251-255
[Patent Document 1] European Patent No. 801067
[Patent Document 2] International Patent Publication WO 2003/6019
[Patent Document 3] Japanese Patent Application Laid-Open Publication No. 2002-104968

### [Disclosure of the Invention]

### [Problems to be Solved by the Invention]

Under such circumstances, development of drugs for neurogenic bladder that have less risk of developing adverse drug reactions and that can be used safely and easily, especially those that can be used as an oral preparation, is desired.

### [Means for Solving the Problems]

The present inventors have keenly studied agents that alleviate urinary urgency, pollakiuria and urinary incontinence and found that solifenacin succinate that has been used as a therapeutic agent for urinary urgency, pollakiuria and urinary incontinence associated with overactive bladder, that is, unstable bladder can improve these symptoms in neurogenic bladder unexpectedly at a dose of 5 mg to 10 mg as solifenacin succinate (3.8 mg to 7.5 mg, respectively, as a free base of solifenacin), which dose is the same as the optimal dose for the treatment of unstable bladder, and completed the present invention.

That is, according to the present invention, an agent for improvement of urinary urgency, pollakiuria or urinary incontinence due to neurogenic bladder comprising (a) solifenacin succinate in an amount of 5 mg to 10 mg or (b) solifenacin or a pharmaceutically acceptable salt thereof in an amount equimolar to 5 mg to 10 mg of solifenacin succinate as an active ingredient as a daily dose is provided; and particularly the above agent for improvement for oral administration; and further the above agent for improvement that is used for adult patients are provided.
Among others, the above pharmaceutical agent that is an agent for improvement of urinary urgency due to neurogenic bladder is preferable; and as another aspect, the above pharmaceutical agent that is an agent for improvement of pollakiuria due to neurogenic bladder is preferable; and as further another aspect, the above pharmaceutical agent that is an agent for improvement of urinary incontinence due to neurogenic bladder is preferable.
Further, among the above agents for improvement, the above agent for improvement comprising (a) solifenacin succinate in an amount of 5 mg or (b) solifenacin or a pharmaceutically acceptable salt thereof in an amount equimolar to 5 mg of solifenacin succinate as an active ingredient as a daily dose is preferable; and as another aspect, the above agent for improvement comprising (a) solifenacin succinate in an amount of 10 mg or (b) solifenacin or a pharmaceutically acceptable salt thereof in an amount equimolar to 10 mg of solifenacin succinate as an active ingredient as a daily dose is preferable.

In addition, according to the present invention, there is provided use of (a) solifenacin succinate in an amount of 5 mg to 10 mg or (b) solifenacin or a pharmaceutically acceptable salt thereof in an amount equimolar to 5 mg to 10 mg of solifenacin succinate as a daily dose for manufacturing an agent for improvement of urinary urgency, pollakiuria or urinary incontinence due to neurogenic bladder; especially the above use wherein the agent for improvement is an agent for improvement for oral administration; and further the above use wherein the agent for improvement is used for adult patients.
Among others, the above use wherein the agent for improvement is an agent for improvement of urinary urgency due to neurogenic bladder is preferable; and as another aspect, the above use wherein the agent for improvement is an agent for improvement of pollakiuria due to neurogenic bladder is preferable; and as further another aspect, the above use wherein the agent for improvement is an agent for improvement of urinary incontinence due to neurogenic bladder is preferable.
Further, among the above uses for manufacturing an agent for improvement, the use wherein the agent for improvement comprising (a) solifenacin succinate in an amount of 5 mg or (b) solifenacin or a pharmaceutically acceptable salt thereof in an amount equimolar to 5 mg of solifenacin succinate as a daily dose is preferable; and as another aspect, the use wherein the above agent for improvement comprising (a) solifenacin succinate in an amount of 10 mg or (b) solifenacin or a pharmaceutically acceptable salt thereof in an amount equimolar to 10 mg of solifenacin succinate as a daily dose is preferable.

Further, according to the present invention, a method for improvement of urinary urgency, pollakiuria or urinary incontinence due to neurogenic bladder, comprising administering (a) solifenacin succinate in an amount of 5 mg to 10 mg of or (b) solifenacin or a pharmaceutically acceptable salt thereof in an amount equimolar to 5 mg to 10 mg of solifenacin succinate as a daily dose to a patient is provided; especially the method for improvement by oral administration; and further the method for improvement wherein the patient is an adult patient are provided.
Among others, the above method for improvement of urinary urgency due to neurogenic bladder is preferable; and as another aspect, the above method for improvement of pollakiuria due to neurogenic bladder is preferable; and as further another aspect, the above method for improvement of urinary incontinence due to neurogenic bladder is preferable.
Further, among the above methods for improvement, the method for improvement comprising administering (a) solifenacin succinate in an amount of 5 mg or (b) solifenacin or a pharmaceutically acceptable salt thereof in an amount equimolar to 5 mg of solifenacin succinate as a daily dose is preferable; and as another aspect, the method for improvement comprising administering (a) solifenacin succinate in an amount of 10 mg or (b) solifenacin or a pharmaceutically acceptable salt thereof in an amount equimolar to 10 mg of solifenacin succinate as a daily dose is preferable.

Causal diseases of neurogenic bladder in the above agent for improvement of urinary urgency, pollakiuria or urinary incontinence due to neurogenic bladder, in the use for manufacturing an agent for improvement of urinary urgency, pollakiuria or urinary incontinence due to neurogenic bladder, and in the method for improvement of urinary urgency, pollakiuria or urinary incontinence due to neurogenic bladder provided by the present invention include cerebrovascular accidents or cerebral infarction, brain or spinal cord injury due to trauma, multiple sclerosis, Parkinson's disease, peripheral neuropathy, congenital malformation of the nerve system, various spinal lesions, that is, spinal cord compression, injury due to fracture and the like, cervical and lumbar spondylosis, spondylosis deformans, spondylolisthesis, spinal stenosis and the like. According to the present invention, therefore, an agent for improvement of urinary urgency, pollakiuria or urinary incontinence due to neurogenic bladder caused by these diseases, use for manufacturing an agent for improvement of urinary urgency, pollakiuria or urinary incontinence due to neurogenic bladder caused by these diseases, and a method for improvement of urinary urgency, pollakiuria of urinary incontinence due to neurogenic bladder caused by these diseases are provided.

### Effect of the Invention

As described in the Examples below, the pharmaceutical agent according to the present invention is useful as an agent for improvement of urinary urgency, pollakiuria and urinary incontinence due to neurogenic bladder. Especially, as described as background art, although a higher dose is required for an antimuscarinic agent for the treatment of these symptoms in neurogenic bladder than that required for the treatment of these symptoms in unstable bladder, the pharmaceutical agent according to the present invention has a remarkable unexpected effect in that the pharmaceutical agent clearly exerts efficacy against these symptoms in neurogenic bladder at the same dose as that required for the treatment of unstable bladder presenting these symptoms.
As described above, currently, solifenacin succinate is actually used as a therapeutic agent for urinary urgency, pollakiuria and urinary incontinence in patients with unstable bladder, that is, overactive bladder, and can be used to treat urinary urgency, pollakiuria and urinary incontinence in patients with neurogenic bladder at the same dose as the dose for unstable bladder. In addition, since its therapeutic effect is exerted by oral administration, the pharmaceutical agent according to the present invention is an agent for improvement of urinary urgency, pollakiuria and urinary incontinence due to neurogenic bladder that can be used safely and easily, especially an agent for improvement for oral administration.

### Best Mode for Carrying Out the Invention

Solifenacin, the effective ingredient of the pharmaceutical agent of the present invention, can be obtained easily by the method described in Patent Document 1 described above, a method that is self-evident to a person skilled in the art or a variation thereof.

The "salt" in the "solifenacin or a salt thereof" may be any acid addition salt of solifenacin with a pharmaceutically acceptable acid and specifically includes acid addition salts with inorganic acids such as hydrochloric acid, hydrobromic acid, hydroiodic acid, sulfuric acid, nitric acid, phosphoric acid and the like; and acid addition salts with organic salts such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, benzenesulfonic acid, aspartic acid, glutamic acid and the like. Preferably the salt is solifenacin succinate, an acid addition salt with succinic acid.

In addition, since solifenacin has an asymmetric carbon atom, optical isomers based on the asymmetric carbon atom are present. Solifenacin as an active ingredient according to the present invention includes isolates of these optical isomers and a mixture thereof, and preferably
(3R)-quinuclidin-3-yl
(1S)-1-phenyl-1,2,3,4-tetrahydroisoquinoline-2-carboxylate or
(3R)-quinuclidin-3-yl
(1R)-1-phenyl-1,2,3,4-tetrahydroisoquinoline-2-carboxylate, particularly preferably (3R)-quinuclidin-3-yl
(1S)-1-phenyl-1,2,3,4-tetrahydroisoquinoline-2-carboxylate. Accordingly, the most preferable active ingredient of the pharmaceutical agent according to the present invention is a succinate salt of (3R)-quinuclidin-3-yl
(1S)-1-phenyl-1,2,3,4-tetrahydroisoquinoline-2-carboxylate.
Further, the active ingredient of the pharmaceutical agent according to the present invention includes solvates such as ethanolates and ethyl acetate solvate, hydrates and crystalline polymorphs.

The pharmaceutical agent according to the present invention may be prepared using solifenacin or a salt thereof together with pharmaceutical carriers, excipients, and other additives that are routinely used for formulation and according to methods routinely used. Although administration may be conducted by oral administration using tablets, pills, capsules, granules, powders, liquids and the like or by parenteral administration using injections for intravenous injection, intramuscular injection and the like, or suppositories, intranasal, transmucosal, percutaneous administration and the like, and oral administration is preferable.

As a solid composition for oral administration according to the present invention, tablets, powders, granules and the like are used. In such a solid composition, solifenacin or a salt thereof is mixed with at least one inactive diluent, such as lactose; mannitol, glucose, hydroxypropylcellulose, microcrystalline cellulose, starch, polyvinylpyrrolidone, magnesium aluminometasilicate and the like. The composition may contain additives, for example, lubricants such as magnesium stearate, disintegrants such as calcium carboxymethylcellulose, stabilizers, solubilizers and the like, in addition to the inactive diluent, according to ordinary methods. Tablets and pills may be coated with sugar or a gastric or enteric coating film such as sucrose, gelatin, hydroxypropylcellulose, hydroxypropylmethylcellulose phthalate and the like.

A liquid composition for oral administration includes pharmaceutically acceptable emulsions, solutions, suspensions, syrups, elixirs and the like, and contains routinely used inactive diluents, for example, purified water and ethanol. The composition may contain auxiliary agents such as wetting agents and suspending agents, sweeteners, flavors, aromatics and preservatives in addition to the inactive diluents.
The injections for parenteral administration contain sterile aqueous or non-aqueous dissolving agents, suspending agents and emulsifying agents. The aqueous dissolving agents and suspending agents include, for example, distilled water for injection and physiological saline. The non-aqueous dissolving agents and suspending agents include, for example, propylene glycol, polyethylene glycol, vegetable oils such as olive oil, alcohols such as EtOH, Polysolvate 80 and the like. These compositions may further contain auxiliary agents such as preservatives, wetting agents, emulsification agents, dispersing agents, stabilizers, solubilizers and the like. This composition is sterilized, for example, by filtration through a bacterial-retaining filter, blending bactericides or irradiation. Alternatively, a sterile solid composition may be prepared and dissolved in sterile water or a sterile solvent for injection before use.

The pharmaceutical agent according to the present invention may be used in combination with other drugs used for the treatment of urinary tract diseases and neurological diseases simultaneously or at certain intervals. For example, the drugs that can be used in combination with the drug according to the present invention include Tamsulosin, botulinus toxin, interferons, tizanidine, glatiramer, levo-dopa, amantadine and the like.
The pharmaceutical agent according to the present invention is administered at a daily dose of 5 mg to 10 mg as solifenacin succinate or a daily dose equimolar to 5 mg to 10 mg of solifenacin succinate as solifenacin or a salt thereof, although symptoms, disease history, weight, sex, age and the like of a patient may be optionally taken into consideration. Although administration may be conducted by dividing the daily dose into 2 to 4 times, administration once daily is preferable. Administration is preferably oral administration.
The dose of solifenacin as solifenacin succinate or a free base of solifenacin related to the pharmaceutical composition of the present invention is determined by assuming administration to males and females aged 18 years or over. When the pharmaceutical composition is administered to males and females younger than 18 years, it may be administered at a lower dose. Specifically, a half dose, that is, 2.5 mg to 5 mg as solifenacin succinate or 1.9 mg to 3.8 mg as a free base of solifenacin may be administered.

### [Brief Description of Drawings]

Figure 1 is a graph showing changes in bladder capacity in a model rat of pollakiuria associated with neurogenic bladder prepared according to the method in Example 1.
Figure 2 is a graph showing changes in voided volume in a model rat of pollakiuria associated with neurogenic bladder prepared according to the method in Example 1.

### [Examples]

Example 1: Efficacy of Solifenacin Succinate in Rat Model of Pollakiuria due to Cerebral Infarction Efficacy

### 1. Test Method

Male SD rats (270 g to 320 g) were used for the test. After the rats were anesthetized with pentobarbital (50 mg/kg), a vesical fistula cannula and a cannula for drug administration were inserted and indwelled in the bladder and the jugular vein, respectively. A cannula was also inserted and indwelled in the common carotid vein for drug administration. Cerebral infarction was prepared according to the method of Longa et al. (Stroke, 1989, vol. 20, p. 84-91) 2 to 3 days after the surgery. Under halothane anesthesia, the cervical part was incised and a nylon thread was inserted from the common carotid artery to the internal carotid artery and the tip of the thread was carried forward to the origin of the middle cerebral artery and indwelled to induce cerebral ischemia. On the next day, neurological symptoms were observed and scored according to the method of Garcia, et al. (Stroke, 1995, Vol. 26, p. 672-634) and rats having moderate or more severe symptoms (with a score of 13 or less) were selected.
After awakening from anesthesia, the rats were maintained in Bollman cages. The vesical fistula cannula was connected to a syringe pump via a three-way stopcock and physiological saline was continuously infused into the bladder to induce micturition reflex. Another end of the three-way stopcock was connected to a pressure transducer to measure intravesical pressure. After stabilization of micturition reflex, the test drug was administered through the catherter for drug dose inserted in the external carotid vein.

### 2. Evaluation Parameters and Statistical Analysis

The volume of physiological saline required to induce a single micturition reflex at 30 minutes after drug administration, that is, bladder capacity and the voided volume per micturition were measured. The results are shown as mean±standard error.
The Student's t-test was used for a comparison between the sham surgery group and the cerebral infarction group and the Dunnett's multiple comparison test was used for a comparison between the group receiving drug administration and the group not receiving drug administration in the cerebral infarction group.

### 3. Results

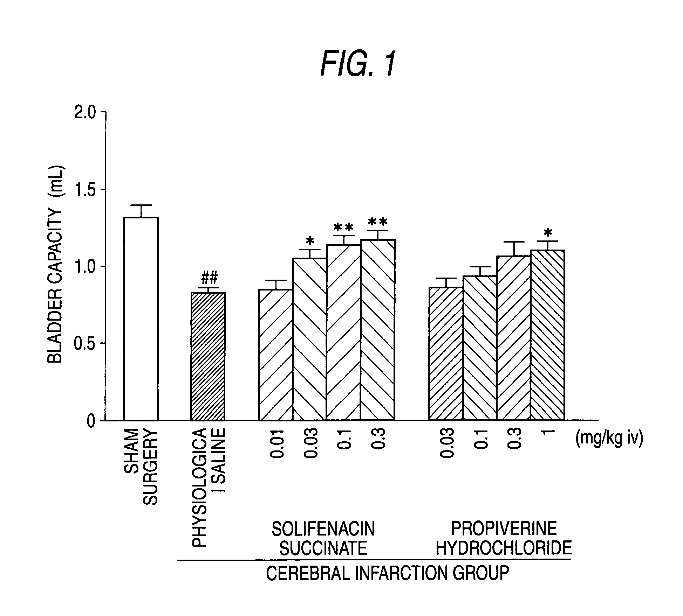

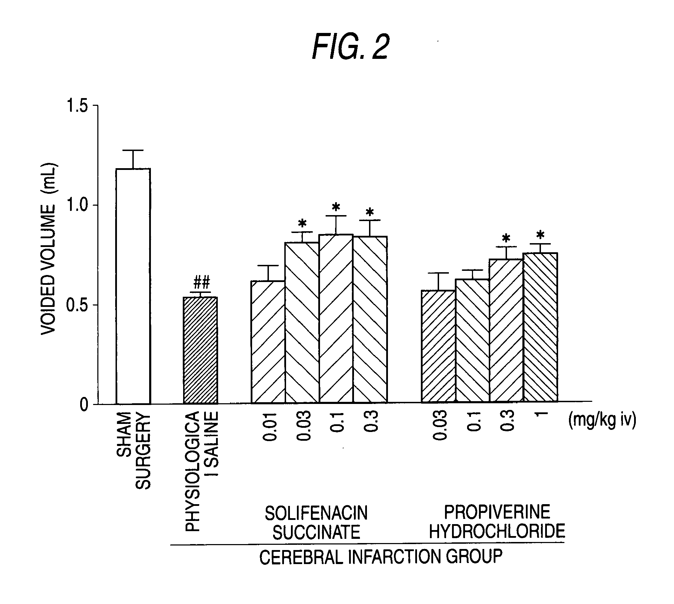

The results are shown in Figures 1 and 2. Preparation of cerebral infarction in the rats decreased the bladder capacity and voided volume. Decreases in bladder capacity and voided volume means that pollakiuria occurs. In this rat model of pollakiuria associated with neurogenic bladder due to cerebral infarction, solifenacin succinate increased the bladder capacity and voided volume dose-dependently at doses of 0.03 mg/kg or higher. In addition, propiverine hydrochloride increased the voided volume at doses 0.3 mg/kg or higher and the bladder capacity at doses of 1 mg/kg or higher. As described above, since solifenacin succinate increased the bladder capacity and voided volume in the rat model of pollakiuria associated with neurogenic bladder in a similar manner to propiverine hydrochloride, solifenacin succinate was considered to be clinically effective as a therapeutic agent for improving pollakiuria due to neurogenic bladder.

### Example 2: Efficacy of Solifenacin Succinate in Humans with Urinary urgency, Pollakiuria and Urinary Incontinence due to Neurogenic Bladder

A clinical study was conducted in patients with urinary urgency, pollakiuria and urinary incontinence under the conditions described below. The causes of urinary urgency, pollakiuria and urinary incontinence were separated into neurogenic bladder (due to cerebral infarction or brain disorder due to brain contusion, peripheral neuropathy, and spinal cord disorder due to cervical or lumbar spondylosis, spondylitis deformans, cervical or lumbar spondylolisthesis, disk herniation, spinal stenosis and the like) and unstable bladder (without accompanying neurological diseases, urinary tract infections and calculi, irradiation history to the lumbar part and tumors in the pelvic organs that can be causal disease of detrussor hyperactivity) and analyses were done.

### 1. Subjects

Male and female patients aged 20 years or older who presented symptoms of urinary urgency, pollakiuria and urinary incontinence for 6 months or a longer period, the number of micturition per 24 hours was 8 on average or higher and satisfied at least one of the following conditions (1) and (2):
(1) Having at least one episode of urge urinary incontinence per 24 hours on average
(2) Having at least one episode of urinary urgency per 24 hours on average. Patients with stress urinary incontinence, psychogenic pollakiuria and the like were excluded. In addition, patients with complication of urinary tract infection, urinary calculi, interstitial cystitis, bladder tumor and the like were also excluded.

### 2. Test' Drugs, Dosage and Administration and Duration of Administration Group P: Placebo (Sham drug)

Group S5: 5 mg of solifenacin succinate (corresponding to 3.8 mg of solifenacin calculated as a free base)
Group S10: 10 mg of solifenacin succinate (corresponding to 7.5 mg of solifenacin calculated as a free base)
Group B: 20 mg of propiverine hydrochloride
All the groups received oral administration once daily for 12 weeks.

### 3. Results

### (1) Mean Change in the Number of Urinary Urgency Episodes

**[Table 1]**

| Mean Change in the Number of Urinary Urgency Episodes in Patients with Neurogenic Bladder and Patients with Unstable Bladder | | | | |
|---|---|---|---|---|
| | Group P | Group S5 | Group S10 | Group B |
| Patients with neurogenic bladder | | | | |
| Number of subjects | 21 | 22 | 21 | 16 |
| Change in the number | -1.83 | -2.86 | -2.43 | -1.65 |
| Difference of change in the number from that in Group P | 0 | -1.03 | -0.60 | +0.18 |

| Patients with unstable bladder | | | | |
|---|---|---|---|---|
| Number of subjects | 374 | 361 | 350 | 368 |
| Change in the number | -1.25 | -2.38 | -2.81 | -2.33 |
| Difference of change in the number from that in Group P | 0 | -1.13 | -1.56 | -1.08 |

As shown in Table 1, efficacy of a similar level was obtained in Group S5 and Group B, and the best result was obtained in the Group S10 in the patients with unstable bladder. In other words, the improving effect on urinary urgency in unstable bladder was observed in all the drug administration groups. For the patients with neurogenic bladder, on the other hand, the improving effect on urinary urgency was observed in the groups receiving the pharmaceutical agent according to the present invention, that is, Group S5 and Group S10, but the improving effect on urinary urgency was not observed in Group B.

### (2) Mean Change in the Number of Micturitions (as Evaluation Parameter for Pollakiuria)

**[Table 2]**

| [0036] Mean Change in the Number of Micturitions in Patients with Neurogenic Bladder and Patients with Unstable Bladder | | | | |
|---|---|---|---|---|
| | Group P | Group S5 | Group S10 | Group B |
| Patients with neurogenic bladder | | | | |
| Number of subjects | 21 | 22 | 21 | 16 |
| Change in the number | -1.48 | -2.04 | -2.59 | -1.25 |
| Difference of change in the number from that in Group P | 0 | -0.56 | -1.11 | +0.23 |

| Patients with unstable bladder | | | | |
|---|---|---|---|---|
| Number of subjects | 374 | 361 | 350 | 368 |
| Change in the number | -0.91 | -1.93 | -2.17 | -1.90 |
| Difference of change in the number from that in Group P | 0 | -1.02 | -1.26 | -0.99 |

As shown in Table 2, efficacy of a similar level was obtained in Group S5 and Group B, and the best result was obtained in the Group S10 in the patients with unstable bladder. In other words, the number of micturitions was decreased in the patients with unstable bladder and the improving effect on pollakiuria in unstable bladder was observed in all drug administration groups. For the patients with neurogenic bladder, on the other hand, the number of mictuiritions was decreased and the improving effect on pollakiuria was observed only in the groups receiving the pharmaceutical agent according to the present invention, that is, Group S5 and Group S10, but the number of micturitions was not decreased and no improving effect on pollakiuria was observed in Group B.

### (3) Mean Change in the Number of Urinary Incontinence Episodes

**[Table 3]**

| [0037] Mean Change in the Number of Urinary Incontinence Episodes in Patients with Neurogenic Bladder and Patients with Unstable Bladder | | | | |
|---|---|---|---|---|
| | Group P | Group S5 | Group S10 | Group B |
| Patients with neurogenic bladder | | | | |
| Number of subjects | 18 | 15 | 14 | 14 |
| Change in the number | -0.61 | -1.80 | -1.40 | -1.62 |
| Difference of change in the number from that in Group P | 0 | -1.19 | -0.79 | -1.01 |

| Patients with unstable bladder | | | | |
|---|---|---|---|---|
| Number of subjects | 265 | 259 | 256 | 281 |
| Change in the number | -0.73 | -1.58 | -1.61 | -1.23 |
| Difference of change in the number from that in Group P | 0 | -0.85 | -0.88 | -0.50 |

As shown in Table 3, the improving effect on urinary incontinence was observed in the patients with unstable bladder in all the drug administration groups. In addition, the improving effect on improving urinary incontinence was also observed in the patients with neurogenic bladder in all the drug administration groups.

### 4. Discussion of the Results

Based on the above results, propiverine hydrochloride, one of the antimuscarinic agents, improved urinary incontinence in the patients with neurogenic bladder at a dose of 20 mg, thereby this dose was confirmed to be the same dose that improved urinary urgency, pollakiuria and urinary incontinence associated with unstable bladder. At this dose, however, propiverine hydrochloride did not possess improving effects on urinary urgency and pollakiuria in the patients with neurogenic bladder, suggesting that a dose higher than 20 mg, that is, a dose higher than the dose required for improving urinary urgency, pollakiuria and urinary incontinence in unstable bladder is required for achieving these improving effects. This findings agree with the report that the dose of oxybutynin, an antimuscarinic agent, required for the treatment of neurogenic bladder is higher than the' dose required for the treatment of unstable bladder as described in the prior art document described above and the description of the package insert of propiverine hydrochloride, and it was confirmed that, generally for antimuscarinic agents, higher doses were required for the treatment of neurogenic bladder than for the treatment of unstable bladder.

On the other hand, the pharmaceutical agents according to the present invention, the pharmaceutical agent containing 5 mg of solifenacin succinate and the pharmaceutical agent containing 10 mg of solifenacin succinate, were shown to improve all the symptoms of urinary urgency, pollakiuria and urinary incontinence in patients with neurogenic bladder. In addition, these doses were the same doses that improve urinary urgency, pollakiuria and urinary incontinence in patients with unstable bladder, and even a clinically recommended dose of 5 mg (half the maximum clinically recommended dose of 10 mg) has proven to be effective in neurogenic bladder.
In other words, under the technical common knowledge that, for antimuscarinic agents, higher doses are generally required for the treatment of urinary urgency, pollakiuria and urinary incontinence in patients with neurogenic bladder than for the treatment of these symptoms in patients with unstable bladder, it was found that the pharmaceutical agent containing solifenacin according to the present invention, the effect of which is completely different from that of antimuscarinic agents, provides therapeutic effect in the treatment of urinary urgency, pollakiuria and urinary incontinence in patients with neurogenic bladder at the same dose as that required for the treatment of these symptoms in patients with unstable bladder. These findings are quite unexpected. In other words, it is considered that since the pharmaceutical agent containing solifenacin according to the present invention exhibits drug properties completely different from the effect of antimuscarinic agents, improvement of urinary urgency, pollakiuria and urinary incontinence was achieved in patients with neurogenic bladder at the same dose that achieves improvement of the variety of symptoms in patients with unstable bladder, that is, without requiring a higher dose than the dose achieving improvement of these symptoms in patients with unstable bladder, by cooperation with any other effect in addition to the effect as an antimuscarinic agent.
Accordingly, the pharmaceutical agent according to the present invention does not require a high dose exceeding the clinically recommended dose for unstable bladder, unlike antimuscarinic agents, and thus can reduce adverse drug reactions, and is considered to be used clinically safely in the treatment of urinary urgency, pollakiuria and urinary incontinence in patients with neurogenic bladder.

### Industrial Applicability

The pharmaceutical agent according to the present invention is useful as an agent for improvement of urinary urgency, pollakiuria and urinary incontinence due to neurogenic bladder caused by neurodegenerative diseases such as cerebrovascular accidents or cerebral infarction, brain or spinal cord injury due to trauma, multiple sclerosis, Parkinson's disease, congenital malformation of the nerve system, peripheral neuropathy, and various spine lesions, that is, spinal cord compression and injury due to fracture, cervical and lumbar spondylosis, spondylosis deformans, spondylolisthesis, spinal stenosis, vertebral disk hernia and the like, especially as an agent for improvement for oral administration.

## Claims

1. An agent for improvement of urinary urgency, pollakiuria or urinary incontinence due to neurogenic bladder, comprising
(a) solifenacin succinate in an amount of 5 mg to 10 mg, or
(b) solifenacin or a pharmaceutically acceptable salt thereof in an amount equimolar to 5 mg to 10 mg of solifenacin succinate
as a daily dose as an active ingredient.

2. The pharmaceutical agent according to claim 1, wherein the pharmaceutical agent is for oral administration.

3. The pharmaceutical agent according to claim 1, wherein the pharmaceutical agent is used for adult patients.

4. The pharmaceutical agent acceding to claim 2, wherein the pharmaceutical agent is used for adult patients.

5. The pharmaceutical agent according to claim 1, 2, 3 or 4, wherein the pharmaceutical agent is an agent for improvement of urinary urgency.

6. The pharmaceutical agent according to claim 1, 2 3 or 4, wherein the pharmaceutical agent is an agent for improvement of pollakiuria.

7. The pharmaceutical agent according to claim 1,2 3 or 4, wherein the pharmaceutical agent is an agent for improvement of urinary incontinence.

8. The pharmaceutical agent according to claim 1, 2, 3 or 4, comprising
(a) solifenacin succinate in an amount of 5 mg, or
(b) solifenacin or a pharmaceutically acceptable salt thereof in an amount equimolar to 5 mg of solifenacin succinate
as an active ingredient.

9. The pharmaceutical agent according to claim 1, 2, 3 or 4 comprising
(a) solifenacin succinate in an amount of 10 mg, or
(b) solifenacin or a pharmaceutically acceptable salt thereof in an amount equimolar to 10 mg of solifenacin succinate
as an active ingredient.

10. Use of
(a) solifenacin succinate in an amount of 5 mg to 10 mg, or
(b) solifenacin or a pharmaceutically acceptable salt thereof in an amount equimolar to 5 mg to 10 mg of solifenacin succinate
as a daily dose for manufacturing an agent for improvement of urinary urgency, pollakiuria or urinary incontinence due to neurogenic bladder.

11. The use according to claim 10, wherein the agent for improvement is an agent for improvement for oral administration.

12. The use according to claim 10, wherein the agent for improvement is an agent for improvement used for adult patients.

13. The use according to claim 11, wherein the agent for improvement is
an agent for improvement used for adult patients.

14. The use according to claim 10, 11, 12 or 13, wherein the agent for improvement is an agent for improvement of urinary urgency.

15. The use according to claim 10, 11, 1 2 or 13, wherein the agent for improvement is an agent for improvement of pollakiuria.

16. The use according to claim 10, 11, 12 or 13, wherein the agent for improvement is an agent for improvement of urinary incontinence.

17. The use according to claim 10, 11, 12 or 13, wherein the use is use of
(a) solifenacin succinate in an amount of 5 mg, or
(b) solifenacin or a pharmaceutically acceptable salt thereof in an amount equimolar to 5 mg of solifenacin succinate.

18. The use according to claim 10, 11, 12 or 13, wherein the use is use of
(a) solifenacin succinate in an amount of 10 mg, or
(b) solifenacin or a pharmaceutically acceptable salt thereof in an amount equimolar to 10 mg of solifenacin succinate.

19. A method for improvement of urinary urgency, pollakiuria or urinary incontinence due to neurogenic bladder, comprising administering
(a) solifenacin succinate in an amount of 5 mg to 10 mg, or
(b) solifenacin or a pharmaceutically acceptable salt thereof in an amount equimolar to 5 mg to 10 mg of solifenacin succinate
as a daily dose to a patient.

20. The method according to claim 19, wherein the administration is oral administration.

21. The method according to claim 19, wherein the patient is an adult patient.

22. The method according to claim 20, wherein the patient is an adult patient.

23. The method according to claim 19, 20, 21 or 22, wherein the method is a method for improvement of urinary urgency.

24. The method according to claim 19, 20, 21 or 22, wherein the method is a method for improvement of pollakiuria.

25. The method according to claim 19, 20, 21 or 22, wherein the method is a method for improvement of urinary incontinence.

26. The method according to claim 19, 20, 21 or 22, wherein the method comprises administering
(a) solifenacin succinate in an amount of 5 mg, or
(b) solifenacin or a pharmaceutically acceptable salt thereof in an amount equimolar to 5 mg of solifenacin succinate.

27. The method according to claim 19, 20, 21 or 22, wherein the method comprises administering
(a) solifenacin succinate in an amount of 10 mg, or
(b) solifenacin or a pharmaceutically acceptable salt thereof in an amount equimolar to 10 mg of solifenacin succinate.
